# EUROPÄISCHE PATENTSCHRIFT

(11) **EP 0 669 309 B1**
(45) Veröffentlichungstag und Bekanntmachung des Hinweises auf die Patenterteilung: **05.11.1997**
(21) Anmeldenummer: 95101915.7
(22) Anmeldetag: 13.02.1995
(51) Int. Cl.: C07C 50/24, C07C 46/06

(54) **Verfahren zur Herstellung von Tetrachlor-1,4-benzochinon**
Process for the preparation of tetrachloro-1,4-benzoquinone
Procédé pour la préparation de la tétrachloro-1,4-benzoquinone

(30) Priorität: 24.02.1994 DE 4405929
(43) Veröffentlichungstag der Anmeldung: 30.08.1995
(73) Patentinhaber: Clariant GmbH, 65929 Frankfurt am Main (DE)
(72) Erfinder: Arndt, Otto, Dr., D-65719 Hofheim (DE); Tronich, Wolfgang, Dr., D-65817 Eppstein (DE)

(56) Entgegenhaltungen:
- EP-A- 0 220 135
- EP-A- 0 278 378

## Beschreibung

Die Erfindung betrifft ein Verfahren zur Herstellung von Tetrachlor-1,4-benzochinon mit hoher Reinheit und verbesserten physikalischen Eigenschaften durch Chlorierung von Hydrochinon mit Chlor in wäßriger Salzsäure und Nachbehandlung der Chlorierungssuspension mit einem organischen Lösungsmittel.

Tetrachlor-1,4-benzochinon (Chloranil) ist ein wertvolles Zwischenprodukt zur Herstellung von Farbstoffen. Ferner wird es als Photochemikalie und Vulkanisationsmittel eingesetzt und dient als Zusatz für Schmiermittel.

Die Herstellung von Chloranil aus Hydrochinon (1,4-Dihydroxy-benzol) oder 1,4-Benzochinon bzw. chloriertem 1,4-Benzochinon ist bekannt.

In der Patentschrift EP 0 220 135 wird z. B. ein Verfahren beschrieben, bei dem Salzsäure und Chlor unter Druck vorgelegt werden und Chlor und Chinon, Hydrochinon oder deren Chlorderivate in getrennten Strömen unter Druck zudosiert werden.

In Patentschrift EP 0 278 378 wird ein Verfahren beschrieben, bei dem die Gesamtmenge des Hydrochinons mit Salzsäure vorgelegt wird und dann Chlor bei Normaldruck nach einem bestimmten Temperatur- und Verdünnungsprogramm eingegast wird.

In der japanischen Patentanmeldung JP 05155804 wird ein Verfahren beschrieben, bei dem Hydrochinon z.B. in einem Gemisch von 25 %iger Salzsäure und einem mit wäßriger Salzsäure nicht mischbarem, jedoch chlorresistenten Lösungsmittel (z.B. o-Dichlorbenzol) mit einem Chlorwasserstoff-Druck von ca. 2 bar bei 80°C in 7 Stunden mit Chlorgas behandelt wird. Die auf Raumtemperatur abgekühlte Suspension wird filtriert und das Chloranil auf dem Filterorgan mit Wasser und schließlich Methanol gewaschen.

Diese Verfahrensweisen weisen Nachteile auf:

Das Verfahren nach EP 0 220 135 verwendet 3 bis 12 bar Chlordruck und erfordert eine exakte synchronisierte Dosierung für die Chlor- und Hydrochinon-Ströme.

Das Verfahren nach EP 0 278 378 hat folgende Nachteile:
1.) Durch das Vorlegen der Gesamtmenge des Hydrochinons bilden sich im Laufe der Reaktion Krusten an der Reaktorwand. Außerdem durchläuft das Reaktionsgemisch Zustände schlechter Rührbarkeit, da es dabei sehr dick wird.
2.) Die langanhaltende thermische Belastung des Reaktionsgemischs bei Temperaturen oberhalb 100°C bewirkt eine Qualitätsverschlechterung durch Spuren Nebenprodukte.
3.) Die lange Nachhlorierungsdauer bedingt ein Verschlechtern der Raumzeitausbeute.

Das Verfahren nach JP 05155804 ist auf Lösemittel beschränkt, die mit Chlor nicht reagieren, außerdem wird das Lösemittel im Volumenverhältnis 1:0,1 - 10, vorzugsweise 1:0,5 - 5 zugesetzt. Dadurch wird die Kesselkapazität und damit auch die Raumzeitausbeute erniedrigt. Aus den in der Beschreibung genannten Lösungsmitteln lassen sich die darin gelösten polychlorierten und toxikologisch bedenklichen Nebenkomponenten (Pentachlorphenol etc.) nur schlecht auf chemischem Wege (z.B. durch Spalten der C-Cl-Bindung bei Reaktion mit Natriumglykolaten) entsorgen. Im Destillationsrückstand verbleibende chlorierte organische Lösemittel verbrauchen zusätzlich Spaltmittel und ergeben Probleme wegen der Verklumpung des Spaltgemischs. Das in der japanischen Anmeldung auch genannte Nitrobenzol bewirkt bei den für die Spaltung notwendigen hohen Temperaturen von ca. 200°C ein erhöhtes Risiko durch Steigerung des thermischen Zersetzungspotentials.

Eine Entsorgung der bei diesem Verfahren anfallenden organischen Lösungsmittel erfordert wegen der Dioxin-Problematik besondere technische Maßnahmen.

Es besteht daher ein Bedarf nach einem einfachen und technisch leicht durchführbaren Verfahren zur Herstellung von Tetrachlor-1,4-benzochinon, das die vorstehend genannten Nachteile vermeidet und Tetrachlor-1,4-benzochinon nicht nur in hoher Reinheit, in hoher Ausbeute und Raumzeitausbeute und hoher Schüttdichte zugänglich macht sondern auch die einfache und kostengünstige Entsorgung der toxikologisch bedenklichen chlorhaltigen Nebenprodukte ermöglicht.

Diese Aufgabe wird gelöst durch ein Verfahren zur Herstellung von Tetrachlor-1,4-benzochinon hoher Reinheit durch Einwirkung von Chlor und konzentrierter Salzsäure auf Hydrochinon, dadurch gekennzeichnet, daß man einen Teil des einzusetzenden Hydrochinons in vorgelegte, katalytische Mengen Eisen(III)-Ionen und ein anionisches Dispergiermittel enthaltende Salzsäure einträgt, in diese Lösung bei einer Temperatur von 20 bis 107°C Chlorgas einleitet, anschließend die restliche Menge Hydrochinon als Feststoff oder gelöst zugibt, und unter weiterem Chloreingasen die Temperatur bis auf 80 bis 107°C steigert, nach Abschluß des Chloreingasens ein höher siedendes, mit Salzsäure nicht mischbares organisches Lösungsmittel zusetzt und anschließend das Reaktionsgemisch einer thermischen Nachbehandlung unterzieht.

Vorteilhaft wird das organische Lösungsmittel in einer Menge von 0,05 bis 2 Teile bezogen auf 1 Teil Tetrachlor-1,4-benzochinon zugesetzt.

Bewährt hat sich die Vorgehensweise, daß man einen Teil des einzusetzenden Hydrochinons in vorgelegte, katalytische Mengen Eisen(III)-Ionen und ein anionisches Dispergiermittel enthaltende 4 bis 6 fach molare Menge - bezogen auf die Gesamtmenge Hydrochinon - 20 bis 37 % wäßrige Salzssäure einträgt, in diese Lösung bei einer Temperatur von 20 bis 90°C die 1,5 bis 2,0 fach molare Menge - bezogen auf Gesamthydrochinon - Chlorgas einleitet, anschließend die restliche Menge Hydrochinon als Feststoff oder gelöst zugibt, die 1,5 bis 2,0 fach molare Chlorgasmenge einleitet, hierbei durch Zugabe von Wasser die Konzentration der Salzsäure zunächst bei 25 bis 31 % hält, unter weiterem Chloreingasen (1,3 bis 1,9 fach molare Menge) und Verdünnen mit Wasser bis zu einer Salzsäurekonzentration von 20 bis 22 % die Temperatur bis auf 100 bis 107°C steigert, nach Abschluß des Chloreingasens ein höher siedendes, mit Salzsäure nicht mischbares organisches Lösemittel in einer Menge von 0,15 bis 1,0 Teilen bezogen auf 1 Teil Tetrachlor-1,4-benzochinon bzw. in einer Menge von ca. 2 - 10 Gew.-% bezogen auf das Reaktionsgemisch zusetzt und anschließend das Reaktionsgemisch einer thermischen Nachbehandlung unterzieht.

In vielen Fällen hat es sich bewährt, 22 bis 33 insbesondere 31 %ige Salzsäure bzw. an HCl ca. 20 bis 22 % enthaltende Mutterlauge (Recyclingsäure) einzusetzen und die erste Phase der Chlorierung bei einer Temperatur von 40 bis 90°C, insbesondere 50 bis 80°C durchzuführen. Es hat sich als vorteilhaft erwiesen, 30 bis 70 %, insbesondere 40 bis 60 %, bevorzugt 45 bis 55 % des einzusetzenden Hydrochinons vorzulegen und die Konzentration der Salzsäure auf 22 bis 28 %, insbesondere 23 bis 25 %, einzustellen.

Die Chlorierung läuft vorteilhaft in 5 bis 15 Stunden, insbesondere 8,5 bis 10,5 Stunden ab.

Es hat sich als günstig erwiesen, das mit Salzsäure nicht mischbare organische Lösungsmittel in einer Menge von 0,2 bis 0,5 Teilen bezogen auf 1 Teil Tetrachlorbenzochinon oder in einer Menge von 2 bis 4 Gew.-% bezogen auf das Reaktionsgemisch zuzusetzen.

Als Lösungsmittel haben sich aromatische Kohlenwasserstoffe, insbesondere Xylol, Toluol oder Diisopropylnaphthalin bewährt; grundsätzlich sind natürlich auch halogenierte Aromaten, wie z.B. Chlorbenzol einsetzbar, jedoch haben diese aus den oben aufgeführten Gründen keine Vorteile.

Die thermische Nachbehandlung wird vorteilhaft bei 90 bis 100°C und Normaldruck durchgeführt.

Durch diese Verfahrensweise lassen sich gegenüber EP 0 278 378 überraschenderweise wesentliche Verbesserungen von Raumzeitausbeute und Produktqualität erreichen. Die Verfahrensänderung besteht darin, daß nicht mehr die Gesamtmenge des Hydrochinons vorgelegt wird, sondern daß das Hydrochinon in zwei Anteilen dem Ansatz zugegeben wird: der erste Teil als Vorlage zusammen mit der Salzsäure, der zweite Teil als Feststoff oder in wäßriger ca. 10 bis 20 %iger, insbesondere 15-18 %iger Lösung in einmaliger Zugabe bei erhöhter Temperatur nach dem ersten Einleiten von Chlorgas. Dabei läuft Wasser in konstantem Strom zu, so daß die Konzentration der Salzsäure auf ca. 25 bis 31 % gehalten wird. Nach Ablauf der ersten Chlorierungsstufe wird weiter Chlor eingegast, jedoch nun unter Temperaturerhöhung bis nahe an den Siedepunkt der azeotropen Salzsäure (ca. 105 bis 107°C), deren Konzentration von ca. 20 bis ca. 22 % durch Wasserzugabe eingestellt wird.

Die Verfahrensänderung gegenüber JP 5 155 804 besteht darin, daß das organische Lösemittel erst nach der Chlorierung zugesetzt wird und zwar in wesentlich geringerer, - sozusagen "katalytischer Menge" (z.B. ca. 2 - 4 Gew.-% bezogen auf das Reaktionsgemisch).

Die Vorteile dieser Verfahrensweise gegenüber EP 0 278 378 sind:
Die technische Durchführbarkeit der Reaktion wird verbessert:
Die Rührbarkeit wird erleichtert trotz verringertem Salzsäure-Einsatz, der Wärmeübergang verbessert, die Kühlsole entfällt, die Homogenität des Reaktionsgemischs wird durch den Wegfall der Krusten hergestellt, der Zeitaufwand für die Chlorierung und Oxidation wird erniedrigt, die Einsatzmenge des Chlorgases und damit auch die Abgasbelastung an Chlor wird verringert, die Qualität des Produkts (ersichtlich an weniger Komponenten im HPLC) und ihre Reproduzierbarkeit werden verbessert.

Die Vorteile dieser Verfahrensweise gegenüber der japanischen Anmeldung JP 05155804 sind:
Das organische Lösungsmittel wird in nur sehr geringer, sozusagen "katalytischer" Menge zugesetzt. Dadurch wird eine bessere Ausnutzung der Kesselkapazität erzielt. Das organische Lösungsmittel muß nicht chlor-resistent sein, wodurch sich die Palette der verfügbaren Lösemittel erweitert. Die Erweiterung der Lösungsmittel-Palette ermöglicht "maßgeschneiderte" Verfahrensbedingungen bei der Entsorgung der polychlorierten Nebenkomponenten durch Verbrennung oder chemische Spaltung.

Das nach der Nachbehandlung ("Formierung") mit organischem Lösungsmittel aus dem Reaktionsgemisch durch Filtration isolierte feuchte Chloranil-Filtergut enthält Wasser und "Formierungsmittel". Beide Anteile werden z.B. mit einem Alkohol, bevorzugt Methanol oder Ethanol, herausgewaschen. Aus dem Waschfiltrat kann der Alkohol durch fraktionierte Destillation zurückgewonnen werden. Der Destillationsrückstand enthält die polychlorierten Nebenkomponenten in angereicherter Form, wobei es sich als vorteilhaft erweist, daß wegen der Anwesenheit von organischem Lösemittel der Destillationssumpf rührbar bleibt und somit leicht aus dem Gefäß entfernt werden kann. In dieser Form werden die polychlorierten Nebenkomponenten der Entsorgung zugeführt.

Die Behandlung dieses Rückstands mit dem Natriumsalz des Ethylenglykols und/oder Butylpolyglykol im Gemisch mit Glykol/Tetraethylenglykoldimethylether/evtl. Diisopropylnaphthalin bei Temperaturen von 185 bis 195°C lieferte nach 7 Stunden Spaltungsergebnisse bis zu ca. 97 % der C-Cl-Bindung zu Chlorid.

Bei Verwendung eines chlorhaltigen organischen Lösemittels würde sich ein Anteil davon ebenfalls im Destillationssumpf anreichern und zu einem erhöhten Verbrauch an Spaltmittel (Glykolat) führen.

Das Verfahren ist flexibel in der Temperaturführung und in der Art der Zugabe des zweiten Anteils des Hydrochinons.

Die Gründe für die gegenüber dem zitierten Stand der Technik verbesserte Verfahrensführung und verbesserte Reproduzierbarkeit sind
a) das Arbeiten bei Normaldruck (Aspekte der Sicherheit),
b) die einfacheren Dosierbedingungen (eine vorübergehende Chlorüberdosierung ist unschädlich bezüglich Abgasbelastung, da das Chlor durch das im Überschuß vorliegende Hydrochinon abgepuffert wird). Der 2. Anteil des Hydrochinons kann als Feststoff oder als wäßrige Lösung schnell vor weiterem Chlor-Eingasen oder langsam mit dem weiterem Chlor-Eingasen zugegeben werden.
c) die einfachere Kühltechnik (Kühlung auf höherem Temperaturniveau),
d) die bessere Rührbarkeit der Suspension trotz verringerter Salzsäurevorlage,
e) die geringere Neigung zur Krustenbildung,
f) die Abnahme von Chlor im Abgas,
g) kein Chlorwasserstoff im Abgas,
h) die raschere Oxidation der intermediär gebildeten polychlorierten Chinhydrone bzw. Hydrochinone durch das Chlor,
i) Die geringere thermische Belastung bei der Nachchlorierung,
j) die Unabhängigkeit von der Zusammensetzung der Gasatmosphäre im Reaktor (Luft oder Stickstoff),
k) die konstante, eine Umkristallisation vermeidende Qualität des isolierten Chloranils.
l) das Chloranil wird in größeren Kristallen erhalten und somit eine höhere Schüttdichte erzielt. Außerdem ist die elektrostatische Aufladung verschwunden.

Das erfindungsgemäß hergestellte Chloranil ist von hoher Reinheit, (Schmelzpunkt, HPLC) und hat im Vergleich zu den Angaben in der zitierten EP 0 278 378 weniger Nebenkomponenten bei gleichzeitig erhöhter Raumzeitausbeute.

Außerdem vergrößert die Nachbehandlung mit dem organischen Lösungsmittel (hier als "Formierung" bezeichnet) die mittlere Korngröße erheblich (von ca. 10 bis 20 µm auf ca. 50 bis 100 µm) während sich die "Spannweite" der Korngrößenverteilung deutlich verkleinert (von ca. 2,3 auf 1,3). Unter dem Licht- oder Rasterelektromikroskop läßt sich der Unterschied optisch deutlich machen. Damit sinkt auch das "Stampfvolumen" des Kristallpulvers (bzw. steigen "Schüttgewicht und Rüttgewicht"). Weiterhin ist durch die Verkleinerung der Gesamtoberfläche die elektrostatische Aufladung verschwunden, die ohne Nachbehandlung ("Formierung") zu beobachten ist. Die Rieselfähigkeit wird verbessert und die Staubexplosionsfähigkeit gemindert.

Die nachstehenden Beispiele dienen der Erläuterung des erfindungsgemäßen Verfahrens, ohne es zu beschränken. Teile sind hierbei Gewichtsteile.

### Beispiel 1

### Erste Stufe

Man trägt in 287 Teile Salzsäure 31 % (2,4 Mol), die 0,12 Teile Eisen(III)chlorid als Oxidationskatalysator und 0,50 Teile eines anionischen Dispergiermittels enthält, 27,7 Teile Hydrochinon (0,25 Mol) als Feststoff ein. Man gast bei 70°C in 3 Stunden 125 Teile Chlor (1,76 Mol) ein. Gleichzeitig laufen 250 Teile 70°C heißes Wasser zu. Wenn 50 % des Chlors und des Wassers zugegeben sind, werden weitere 27,7 Teile Hydrochinon (0,25 Mol) als Feststoff in einer einmaligen Portion zugegeben.
Dieser zweite Anteil Hydrochinon kann auch gelöst in 125 Teilen 70°C heißem Wasser zudosiert werden, wobei die Zudosierungsgeschwindigkeit (vorzugsweise 10 Minuten, max. 1,5 Stunden) keine entscheidende Rolle spielt. In diesem Fall wird die Menge des Lösungswassers auf die Menge des zulaufenden Wassers angerechnet, so daß nur 125 Teile Wasser zulaufen statt der oben erwähnten 250 Teile. Man erhält eine gut rührbare, krustenfreie, bräunlichgelbe Suspension. Das Abgas wird überwacht. Es dürfen kein Chlor und kein Chlorwasserstoff in das Abgas gelangen.
Die Konzentration der Salzsäure beträgt ca. 25 %.

### Zweite Stufe

Das Reaktionsgemisch wird unter weiterem Eingasen von 45 Teilen Chlor (= 0,63 Mol) in 3 Stunden auf 90°C geheizt. Die Konzentration der Salzsäure beträgt ca. 28 %. Es bildet sich eine gut rührbare, krustenfreie, hellgelbe Suspension.
Das Abgas wird überwacht. Es darf noch kein Chlor entweichen bzw. ins Abgas gelangen.

### Verdünnen mit Wasser

Man läßt in 15 Minuten 200 Teile Wasser zulaufen. Danach beträgt die HCl-Konzentration ca. 21 %.

### Dritte Stufe

Man heizt die dünne und hellgelbe Suspension in 2 Stunden auf 105°C und gast dabei 12,5 Teile Chlor (= 0,18 Mol) ein.
Das Reaktionsgemisch enthält in diesem Stadium gemäß DC-Analyse weniger als 10 Mol-% Trichlorbenzochinon.

### Vierte Stufe

Die Temperatur des Reaktionsgemischs wird 1,5 Stunden auf 103 - 106°C, bevorzugt 105°C, gehalten. Dabei werden erneut max. 10 Teile = 0,14 Mol Chlor eingegast. Das Reaktionsgemisch enthält weniger als 5 Mol-% Trichlorbenzochinon.

Man arbeitet in allen Stufen im offenen System (Belüftung über Abgasanlage), so daß sich kein Druck aufbauen kann.
Die Chlorierzeit beträgt insgesamt max. 10 Stunden, während dieser Zeit werden insgesamt 192,5 Teile Chlor (2,71 Mol) eingegast.

### Behandeln mit Xylol

Zu der 100°C heißen Produktsuspension läßt man 25 Teile Xylol zulaufen. Nach wenigen Minuten wird die Suspension deutlich dünnflüssiger. An der Gefäßwand durch Verspritzen haftende Produktsuspension wird rasch durch rückfließendes Xylol heruntergespült. Man rührt noch 1 - 2 Stunden bei 100°C nach. Das Reaktionsgemisch enthält jetzt weniger als 1 Mol-% Trichlorbenzochinon. Die Behandlung mit Xylol verbessert zum einen die Qualität des Chloranils, außerdem erfolgt eine wesentliche Vergröberung der Kristalle.

### Isolieren des Chloranils

Man kühlt den Ansatz auf 30°C unter Überlagerung mit Schutzgas (Stickstoff) ab, wodurch ein Zurücksaugen von Chlor aus der Abgasabsorption verhindert wird und die Atmosphäre über dem Reaktionsgemisch entgiftet wird.
Nach Filtration bei 30°C und Waschen mit zunächst 200 Teilen Methanol, dann mit 500 Teilen heißem Wasser erhält man 115 Teile Chloranil mit einem Reingehalt von 99,0 % (= 0,463 Mol), entspr. einer Ausbeute von 92,6 % der Theorie. Verunreinigungen wie Tetrachlorhydrochinon und polychlorierte Chinhydrone bzw. Hydrochinone sind nicht nachweisbar (DC). Der Gehalt an 2,3,5-Trichlorbenzochinon beträgt max. 1,0 Mol-% (HPLC).
Der Gehalt an Pentachlorphenol beträgt 0 bis max. 10 µg/g (HPLC).
Die Korngrößenverteilung (Malvern Laserlichtbeugung, ohne Ultraschallbehandlung) hat ihr Maximum bei 100 µm. Das Produkt ist gut rieselfähig.
Die Mutterlauge ist eine ca. 21 %ige Salzsäure . Das methanolische Waschfiltrat enthält fast die gesamte Menge Xylol. Aus dem methanolischen Waschfiltrat wird das Methanol durch Destillation regeneriert. Der Destillationssumpf kann durch Verbrennen entsorgt werden. Er enthält Pentachlorphenol und andere Nebenkomponenten mit organ. gebundenem Chlor. Diese können selbstverständlich auch auf chemischem Weg z.B. durch Behandeln mit Glykolaten entsorgt werden, wobei die Gegenwart von Xylol, das selbst nicht Chlor enthält, nicht stört.
Die 21 % HCl enthaltende Mutterlauge kann nach Entfernen von sehr geringen Mengen Xylol z. B. durch Aktivkohle einer Wiederverwendung zugeführt werden oder auf üblichem Wege entsorgt werden.
Die Chlorbilanz (Chlorwasserstoff + Chlor, incl. Abgas) beträgt ca. 95 % der Theorie.
Der Chlorumsatz beträgt ca. 99 - 100 % der Theorie.

### Beispiel 2

Man verfährt wie in Beispiel 1, legt jedoch Mutterlauge als Recyclingsäure vor. Anstelle der in Beispiel 1 genannten 287 Teile 31 %iger Salzsäure verwendet man 415 Teile über Aktivkohle geklärte Mutterlauge aus Beispiel 1 (= 50 % der insgesamt angefallenen Mutterlauge) mit einem Gehalt von 87,5 Teilen HCl. Da diese Mutterlauge nun 125 bis 130 Teile zusätzliches Wasser enthält, läßt man von den in Beispiel 1, Erste Stufe, genannten 250 Teilen Wasser nur die zweite Hälfte (ab 1,5 Stunden) bei 70°C zulaufen, bzw. falls, wie in Beispiel 1 beschrieben, der zweite Anteil Hydrochinon als Lösung in 125 Teilen Wasser eingesetzt wird, wird jetzt in der Ersten Stufe kein weiteres Wasser mehr zudosiert.
Nach Filtration bei 30°C und Waschen mit zunächst 200 Teilen Methanol, dann mit 500 Teilen heißem Wasser erhält man 110 Teile Chloranil mit einem Reingehalt von mindestens 99,0 % (= 0,443 Mol), entsprechend einer Ausbeute von 88,6 % der Theorie. Verunreinigungen wie Tetrachlorhydrochinon und polychlorierte Chinhydrone bzw. Hydrochinone sind nicht nachweisbar (DC). Der Gehalt an 2,3,5-Trichlorbenzochinon beträgt max. 1,0 Mol-% (HPLC). Der Gehalt an Pentachlorphenol beträgt 0 bis max. 10 µg/g (HPLC). Die Korngrößenverteilung (Malvern Laserlichtbeugung, ohne Ultraschallbehandlung) hat ihr Maximum bei 100 µm. Das Produkt ist gut rieselfähig.
Die Mutterlauge ist eine ca. 21 %ige Salzsäure. Das methanolische Waschfiltrat enthält fast die gesamte Menge Xylol. Aus dem methanolischen Waschfiltrat wird das Methanol durch Destillation regenereriert. Dabei stört ein Gehalt an Xylol nicht. Der Destillationssumpf kann durch Verbrennen entsorgt werden. Er enthält Pentachlorphenol und andere Nebenkomponenten mit organisch gebundenem Chlor. Diese können selbstverständlich auch auf chemischem Weg z.B. durch Behandeln mit Glykolaten entsorgt werden, wobei die Gegenwart von Xylol, das selbst nicht Chlor enthält, nicht stört. Die 21 % HCl enthaltende Mutterlauge kann nach Klären von sehr geringen Mengen Xylol über z.B. Aktivkohle einer teilweisen Wiederverwendung zugeführt werden oder auf üblichem Wege entsorgt werden.
Die Chlorbilanz (Chlorwasserstoff + Chlor, incl. Abgase) beträgt ca. 95 % der Theorie.
Der Chlorumsatz beträgt ca. 99 - 100 % der Theorie.

### Beispiel 3

Man verfährt wie in Beispiel 1, verwendet jedoch 40 g Xylol für die Formierung der Kristalle und Ethanol für die Wäsche des Chloranils auf der Nutsche.
Die Produktsuspension filtriert man bei 30°C. Man wäscht das Nutschgut bei 25°C mit 200 Teilen Ethanol. Man erhält ein ethanolfeuchtes Chloranil und ein ethanolisches Waschfiltrat (enthält die Hauptmenge des Xylols). Nach dem Waschen mit heißem Wasser und dem Trocknen erhält man 115 Teile Chloranil mit einem Reingehalt von mindestens 99,0 % (= 0,463 Mol), entspr. einer Ausbeute von 92,6 % der Theorie.
Verunreinigungen wie Tetrachlorhydrochinon und polychlorierte Chinhydrone bzw. Hydrochinone sind nicht nachweisbar (DC). Der Gehalt an 2,3,5-Trichlorbenzochinon beträgt max. 1,0 Mol-% (HPLC). Der Gehalt an Pentachlorphenol beträgt < 5 µg/g (HPLC).
Die Korngrößenverteilung (Malvern Laserlichtbeugung, ohne Ultraschallbehandlung) hat ihr Maximum bei 99 µm. Das Produkt ist gut rieselfähig.
Die Mutterlauge ist eine ca. 21 bis 22 %ige Salzsäure . Sie kann nach Entfernen von einer geringen Menge Xylol z. B. durch Aktivkohle einer teilweisen Wiederverwendung zugeführt werden oder auf üblichem Wege entsorgt werden. Aus dem ethanolischen Waschfiltrat wird das Ethanol durch Destillation regeneriert. Der Destillationssumpf kann durch Verbrennen entsorgt werden. Er enthält Pentachlorphenol und andere Nebenkomponenten mit organ.
gebundenem Chlor. Diese können selbstverständlich auch auf chemischem Weg z.B. durch Behandeln mit Glykolaten entsorgt werden, wobei die Gegenwart von Xylol, das selbst nicht Chlor enthält, nicht stört.
Die Chlorbilanz (Chlorwasserstoff + Chlor, incl. Abgas) beträgt ca. 95 % der Theorie.
Der Chlorumsatz beträgt ca. 99 - 100 % der Theorie.

### Beispiel 4

Man verfährt wie in Beispiel 1, verwendet jedoch Xylol sowohl für die Formierung der Kristalle als auch für die Wäsche des Chloranils auf der Nutsche. Die Produktsuspension filtriert man bei 30°C. Man wäscht das Nutschgut bei 25°C mit 100 Teilen Xylol.
Man erhält ein xylolfeuchtes Chloranil (enthält das Xylol aus der Formierung) und ein xylolisches Waschfiltrat. Nach dem Waschen mit heißem Wasser und dem Trocknen erhält man 111,6 Teile Chloranil mit einem Reingehalt von 99,5 % (= 0,452 Mol), entspr. einer Ausbeute von 90,3 % der Theorie. Verunreinigungen wie Tetrachlorhydrochinon und polychlorierte Chinhydrone bzw. Hydrochinone sind nicht nachweisbar (DC). Der Gehalt an 2,3,5-Trichlorbenzochinon beträgt 0,3 Mol-% (HPLC).
Der Gehalt an Pentachlorphenol beträgt kleiner 5 µg/g (HPLC).
Die Korngrößenverteilung, (Malvern Laserlichtbeugung, ohne Ultraschallbehandlung) hat ihr Maximum bei 100 µm. Das Produkt ist gut rieselfähig.
Die Mutterlauge ist eine ca. 22 %ige Salzsäure. Sie kann nach Klären von Spuren Xylol über z. B. Aktivkohle einer teilweisen Wiederverwendung zugeführt werden oder auf üblichem Wege entsorgt werden.
Aus dem xylolischen Waschfiltrat wird das Xylol durch Destillation regeneriert. Der Destillationssumpf kann durch Verbrennen entsorgt werden. Er enthält Pentachlorphenol und andere Nebenkomponenten mit organ. gebundenem Chlor. Diese können selbstverständlich auch auf chemischem Weg z.B. durch Behandeln mit Glykolaten entsorgt werden, wobei die Gegenwart von Xylol, das selbst nicht Chlor enthält, nicht stört.
Die Chlorbilanz (Chlorwasserstoff + Chlor, incl. Abgas) beträgt ca. 98 % der Theorie.
Der Chlorumsatz beträgt ca. 99 - 100 % der Theorie.

### Beispiel 5

Man verfährt wie in Beispiel 1, verwendet jedoch Chlorbenzol sowohl für die Formierung der Kristalle als auch für die Wäsche des Chloranils auf der Nutsche. Schon nach 20 Teilen Chlorbenzol bei 100°C wird die Suspension deutlich dünnflüssiger. An der Reaktorwand haftende Produktsuspension wird in das Reaktionsgemisch herunter gespült. Man führt die Formierung der Kristalle mit 40 Teilen Chlorbenzol (auf ca. 1100 Teile Reaktionsgemisch) bei 100°C in 2 Stunden durch.
Man filtriert das Chloranil aus dem Reaktionsgemisch bei 90°C.
Das Chlorbenzol (aus der Kristallformierung) verbleibt quantitativ im Nutschgut. Man wäscht das Nutschgut bei 25°C mit 200 Teilen Chlorbenzol.
Man erhält ca. 150 bis 155 Teile chlorbenzolfeuchtes Chloranil und ein chlorbenzolisches Waschfiltrat, das zur Regeneration des Chlorbenzols und Entsorgung der darin enthaltenen polychlorierten Nebenkomponenten geht. Aus dem chlorbenzolfeuchten Chloranil wird das restliche Chlorbenzol mit Wasserdampf ausgetrieben (= ca. 10 bis 20 Teile). Die chlorbenzolfreie wäßrige Suspension wird bei 90°C filtriert und mit 500 Teilen heißem Wasser gewaschen.
Man erhält 112 Teile Chloranil mit einem Reingehalt von mindestens 99,0 % (= 0,451 Mol), entspr. einer Ausbeute von 90,2 der Theorie.
Verunreinigungen wie Tetrachlorhydrochinon und polychlorierte Chinhydrone bzw. Hydrochinone sind nicht nachweisbar (DC). Der Gehalt an 2,3,5-Trichlorbenzochinon beträgt 0,3 bis max.
1,0 Mol-% (HPLC). Der Gehalt an Pentachlorphenol beträgt 0 bis max. 5 µg/g (HPLC).
Die Korngrößenverteilung (Malvern Laserlichtbeugung, ohne Ultraschallbehandlung) hat ihr Maximum bei 51 µm. Das Produkt ist gut rieselfähig.
Die Mutterlauge ist eine ca. 20 bis 21 %ige Salzsäure. Sie kann nach Klären von sehr geringen Mengen Chlorbenzol über z. B. Aktivkohle einer teilweisen Wiederverwendung zugeführt werden oder auf üblichem Wege entsorgt werden.

Aus dem chlorbenzolischem Waschfiltrat wird das Chlorbenzol durch Destillation regeneriert. Der Destillationssumpf enthält Pentachlorphenol und andere Nebenkomponenten mit organ. gebundenem Chlor. Diese können selbstverständlich auf chemischem Weg z.B. durch Behandeln mit Glykolaten entsorgt werden, wobei jedoch restliches Chlorbenzol zunächst durch Wasserdampfdestillation entfernt werden muß.
Die Chlorbilanz (Chlorwasserstoff + Chlor, incl. Abgas) beträgt ca. 95 % der Theorie.
Der Chlorumsatz beträgt ca. 99 - 100 % der Theorie.

### Beispiel 6

Man verfährt wie in Beispiel 1, verwendet jedoch nun als "Formierungsmittel" Toluol statt Xylol und zur Wäsche des Chloranils auf der Nutsche Toluol und Ethanol statt Methanol.
Nach Filtration bei 30°C und Waschen mit zunächst 50 Teilen Toluol, dann mit 200 Teilen Methanol und schließlich mit 500 Teilen heißem Wasser erhält man 113 Teile Chloranil mit einem Reingehalt von mindestens 99,0 % (= 0,455 Mol), entsprechen einer Ausbeute von 91,0 % der Theorie. Verunreinigungen wie Tetrachlorhydrochinon und polychlorierte Chinhydrone bzw. Hydrochinone sind nicht nachweisbar (DC). Der Gehalt an 2,3,5-Trichlorbenzochinon beträgt 0,4 Mol-% (HPLC).
Der Gehalt an Pentachlorphenol beträgt 0 bis max. 10 µg/g (HPLC).
Die Korngrößenverteilung (Malvern Laserlichtbeugung, ohne Ultraschallbehandlung) hat ihr Maximum bei 100 µm. Das Produkt ist gut rieselfähig.
Die Mutterlauge ist eine ca. 22 %ige Salzsäure. Das ethanolsiche Waschfiltrat enthält fast die gesamte Menge Toluol. Aus dem ethanolischen Waschfiltrat wird das Ethanol durch Destillation regeneriert. Dabei stört ein Gehalt an Toluol nicht. Der Destillationssumpf kann durch Verbrennen entsorgt werden. Er enthält Pentachlorphenol und andere Nebenkomponenten mit organisch gebundenem Chlor. Diese können selbstverständlich auch auf chemischem Weg z.B. durch Behandeln mit Glykolaten entsorgt werden, wobei die Gegenwart von Toluol, das selbst nicht Chlor enthält, nicht stört. Die 22 % HCl enthaltende Mutterlauge kann nach Klären von sehr geringen Mengen Toluol über z.B. Aktivkohle einer teilweisen Wiederverwendung zugeführt werden oder auf üblichem Wege entsorgt werden.
Die Chlorbilanz (Chlorwasserstoff + Chlor, incl. Abgas) beträgt ca. 95 % der Theorie.
Der Chlorumsatz beträgt ca. 99 bis 100 % der Theorie.

### Beispiel 7

### Vergleichsbeispiel:

### (Ausführung ohne Anwendung von organischem Lösemittel)

Man verfährt wie in Beispiel 1, verwendet jedoch kein organisches Lösemittel weder zur Formierung der Kristalle noch zur Wäsche des Chloranils auf der Nutsche.
Man erhält 119 Teile Chloranil mit einem Reingehalt von 97,0 % (= 0,469 Mol), entspr. einer Ausbeute von 93,9 % der Theorie.
Verunreinigungen wie Tetrachlorhydrochinon und polychlorierte Chinhydrone bzw. Hydrochinone sind nicht nachweisbar (DC). Der Gehalt an 2,3,5-Trichlorbenzochinon beträgt 2,5 bis 3,0 Mol-% (HPLC). Der Gehalt an Pentachlorphenol beträgt 220 µg/g (HPLC).
Die Korngrößenverteilung (Malvern Laserlichtbeugung, ohne Ultraschallbehandlung] hat ihr Maximum bei 12 µm. Das Produkt ist schlecht rieselfähig und elektrostatisch aufgeladen.
Die Mutterlauge ist eine ca. 20 bis 21 %ige Salzsäure. Die Chlorbilanz (Chlorwasserstoff + Chlor, incl. Abgas) beträgt ca. 97 % der Theorie. Der Chlorumsatz beträgt ca. 99 - 100 % der Theorie.

### Beispiel 8

### (Vergleichsbeispiel, aus Ciba-Patentschrift):

siehe EP 0220135, Beispiel 2

### Beispiel 9

### (Vergleichsbeispiel, aus japan. Offenlegung 5 155 804, Ausführungsbeispiel 1)

Ein Glasautoklav wird mit 400 ml o-Dichlorbenzol, 400 ml 25 %iger Salzsäure und 81,6 g Hydrochinon (0,74 Mol) beschickt. Das Gemisch wird auf 80°C erwärmt. Dann werden über 7 Stunden 288,6 g Chlorgas eingeleitet. Es wird 1 Stunde nachgerührt. Mit dem Reaktionsfortschritt erhöht sich der Druck im Autoklaven. Sobald ein Druck von 2 kg/cm² (nach 2 Mol Chlor bezogen auf 1 Mol Hydrochinon) erreicht ist, wird dieser Druck bis zum Reaktionsende durch ein begrenzendes Steuerventil in der Abgasleitung konstant gehalten.
Nach Abkühlen auf Raumtemperatur wird das Kristallisat über eine Glasnutsche filtriert, dreimal mit je 200 ml Wasser und dreimal mit je 200 ml Methanol gewaschen.
Man erhält 178,5 g Chloranil (Ausbeute = 98,1 %). Der Reingehalt beträgt gemäß GC - Analyse 99,85 % (Trichlorbenzochinon = 0,15 %).

## Patentansprüche

1. Verfahren zur Herstellung von Tetrachlor-1,4-benzochinon durch Einwirkung von Chlor und konzentrierter Salzsäure auf Hydrochinon, dadurch gekennzeichnet, daß man einen Teil des einzusetzenden Hydrochinons in vorgelegte, katalytische Mengen Eisen(III)-Ionen und ein anionisches Dispergiermittel enthaltende Salzssäure einträgt, in diese Lösung bei einer Temperatur von 20 bis 107°C Chlorgas einleitet, anschließend die restliche Menge Hydrochinon als Feststoff oder gelöst zugibt, unter weiterem Chloreingasen die Temperatur bis auf 80 bis 107°C steigert, nach Abschluß des Chloreingasens ein höher siedendes, mit Salzsäure nicht mischbares organisches Lösungsmittel zusetzt und anschließend das Reaktionsgemisch einer thermischen Nachbehandlung unterzieht.

2. Verfahren nach Anspruch 1, dadurch gekennzeichnet, daß man das organische Lösungsmittel in einer Menge von 0,05 bis 2 Teile bezogen auf 1 Teil Tetrachlor-1,4-benzochinon zusetzt.

3. Verfahren nach Anspruch 1 oder 2, dadurch gekennzeichnet, daß man einen Teil des einzusetzenden Hydrochinons in vorgelegte, katalytische Mengen Eisen(III)-Ionen und ein anionisches Dispergiermittel enthaltende 4 bis 6 fach molare Menge - bezogen auf die Gesamtmenge Hydrochinon - 20 bis 37 % wäßrige Salzssäure einträgt, in diese Lösung bei einer Temperatur von 20 bis 90°C die 1,5 bis 2,0 fach molare Menge - bezogen auf Gesamthydrochinon - Chlorgas einleitet, anschließend die restliche Menge Hydrochinon als Feststoff oder gelöst zugibt, die 1,5 bis 2,0 fach molare Chlorgasmenge einleitet, hierbei durch Zugabe von Wasser die Konzentration der Salzsäure zunächst bei 25 bis 31 % hält, unter weiterem Chloreingasen (1,3 bis 1,9 fach molare Menge) und Verdünnen mit Wasser bis zu einer Salzsäurekonzentration von 20 bis 22 % die Temperatur bis auf 100 bis 107°C steigert, nach Abschluß des Chloreingasens ein höher siedendes, mit Salzsäure nicht mischbares organisches Lösemittel in einer Menge von 0,15 bis 1,0 Teilen bezogen auf 1 Teil Tetrachlor-1,4-benzochinon oder in einer Menge von ca. 1 - 10 Gew.-% bezogen auf das Reaktionsgemisch zusetzt und anschließend das Reaktionsgemisch einer thermischen Nachbehandlung unterzieht.

4. Verfahren nach mindestens einem der Ansprüche 1 bis 3, dadurch gekennzeichnet, daß man 30 bis 70 %, insbesondere 40 bis 60 %, bevorzugt 45 bis 55 % des einzusetzenden Hydrochinons vorlegt.

5. Verfahren nach mindestens einem der Ansprüche 1 bis 4, dadurch gekennzeichnet, daß eine 22 bis 33 %ige, insbesondere 31 %ige wäßrige Salzsäure eingesetzt wird.

6. Verfahren nach mindestens einem der Ansprüche 1 bis 4, dadurch gekennzeichnet, daß man eine ca. 20 - 22 %ige Recyclingsäure einsetzt.

7. Verfahren nach mindestens einem der Ansprüche 1 bis 6, dadurch gekennzeichnet, daß bei einer Temperatur von 40 bis 90°C, insbesondere 50 bis 80°C Chlor eingegast wird.

8. Verfahren nach mindestens einem der Ansprüche 1 bis 7, dadurch gekennzeichnet, daß die restliche Menge des Hydrochinons als 10 bis 20 %ige, insbesondere 15-18 %ige wäßrige Lösung zugegeben wird.

9. Verfahren nach mindestens einem der Ansprüche 1 bis 8, dadurch gekennzeichnet, daß die Chlorierung in 5 bis 15 Stunden, insbesondere 8,5 bis 10,5 Stunden durchgeführt wird.

10. Verfahren nach mindestens einem der Ansprüche 1 bis 9, dadurch gekennzeichnet, daß man nach Abschluß des Chloreingasens das mit Salzsäure nicht mischbare organische Lösungsmittel in einer Menge von 0,2 bis 0,5 Teilen bezogen auf 1 Teil Tetrachlorbenzochinon oder in einer Menge von 2 bis 4 Gew.-% bezogen auf das Reaktionsgemisch zusetzt.

11. Verfahren nach mindestens einem der Ansprüche 1 bis 10, dadurch gekennzeichnet, daß als Lösungsmittel ein aromatischer Kohlenwasserstoff, insbesondere Xylol, Toluol oder Diisopropylnaphthalin eingesetzt wird.

12. Verfahren nach mindestens einem der Ansprüche 1 bis 11, dadurch gekennzeichnet, daß die thermische Nachbehandlung bei 90 bis 100°C unter Normaldruck durchgeführt wird.

## Claims

1. A process for the preparation of tetrachloro-1,4-benzoquinone by the action of chlorine and concentrated hydrochloric acid on hydroquinone, which comprises introducing a portion of the hydroquinone to be employed into initially introduced hydrochloric acid containing catalytic amounts of iron(III) ions and an anionic dispersant, introducing chlorine gas into this solution at a temperature of from 20 to 107°C, then adding the residual quantity of hydroquinone as a solid or in solution, raising the temperature to from 80 to 107°C while continuing to pass in gaseous chlorine, adding, after the introduction of gaseous chlorine has ended, a relatively high-boiling organic solvent which is not miscible with hydrochloric acid, and then subjecting the reaction mixture to thermal aftertreatment.

2. The process as claimed in claim 1, wherein the organic solvent is added in a quantity of from 0.05 to 2 parts per part of techrachloro-1,4-benzoquinone.

3. The process as claimed in slain 1 or 2, which comprises introducing a portion of the hydroquinone to be employed into an initially introduced 4- to 6-fold molar quantity - based on the total quantity of hydroquinone - of from 20 to 37% strength aqueous hydrochloric acid containing catalytic amounts of iron(III) ions and an anionic dispersant, introducing from 1.5 to 2.0 times the molar quantity - based on total hydroquinone - of chlorine gas into this solution at a temperature of from 20 to 90°C, then adding the residual quantity of hydroquinone as a solid or in solution, introducing from 1.5 to 2.0 times the molar quantity of chlorine gas, while keeping the concentration of the hydrochloric acid initially at from 25 to 31% by addition of water, raising the temperature to from 100 to 107°C while continuing to pass in gaseous chlorine (from 1.3 to 1.9 times the molar quantity) and while diluting with water to a hydrochloric acid concentration of from 20 to 22%, adding, after the introduction of gaseous chlorine has ended, a relatively high-boiling organic solvent which is not miscible with hydrochloric acid, in a quantity of from 0.15 to 1.0 part per part of tetrachloro-1,4-benzoquinone or in a quantity of about 1 - 10% by weight, based on the reaction mixture, and then subjecting the reaction mixture to thermal aftertreatment.

4. The process as claimed in at least one of claims 1 to 3, wherein from 30 to 70%, in particular from 40 to 60% and preferably from 45 to 55% of the hydroquinone to be employed is initially introduced.

5. The process as claimed in at least one of claims 1 to 4, wherein a from 22 to 33% strength, in particular 31% strength aqueous hydrochloric acid is employed.

6. The process as claimed in at least one of claims 1 to 4, wherein a recycled acid whose concentration is about 20-22% is employed.

7. The process as claimed in at least one of claims 1 to 6, wherein gaseous chlorine is introduced at a temperature of from 40 to 90°C, in particular from 50 to 80°C.

8. The process as claimed in at least one of claims 1 to 7, wherein the residual quantity of the hydroquinone is added in the form of a from 10 to 20% strength, in particular 15-18% strength, aqueous solution.

9. The process as claimed in at least one of claims 1 to 8, wherein the chlorination is carried out over from 5 to 15 hours, in particular from 8.5 to 10.5 hours.

10. The process as claimed in at least one of claims 1 to 9, wherein, after the introduction of gaseous chlorine has ended, the organic solvent which is not miscible with hydrochloric acid is added in a quantity of from 0.2 to 0.5 part per part of tetrachlorobenzoquinone or in a quantity of from 2 to 4% by weight, based on the reaction mixture.

11. The process as claimed in at least one of claims 1 to 10, wherein the solvent employed is an aromatic hydrocarbon, especially xylene, toluene or diisopropylnaphthalene.

12. The process as claimed in at least one of claims 1 to 11, wherein the thermal aftertreatment is carried out at from 90 to 100°C under atmospheric pressure.

## Revendications

1. Procédé pour préparer la tétrachloro-1,4-benzoquinone, sous l'action du chlore et de l'acide chlorhydrique concentré sur l'hydroquinone, caractérisé en ce qu'on introduit une partie de l'hydroquinone à utiliser dans de l'acide chlorhydrique déjà en place, contenant des quantités catalytiques d'ions fer(III) et un dispersant anionique, on introduit dans cette solution du chlore gazeux à une température de 20 à 107°C, puis on ajoute la quantité restante d'hydroquinone sous forme solide ou dissoute, et, tout en poursuivant l'insufflation de chlore, on élève la température à une valeur de 80 à 107°C, et, après la fin de l'insufflation de chlore, on ajoute un solvant organique ayant un point d'ébullition plus haut et non-miscible à l'acide chlorhydrique, puis on soumet le mélange réactionnel à un post-traitement thermique.

2. Procédé selon la revendication 1, caractérisé en ce qu'on ajoute le solvant organique en une quantité de 0,05 à 2 parties par partie de tétrachloro-1,4-benzoquinone.

3. Procédé selon la revendication 1 ou 2, caractérisé en ce qu'on introduit une partie de l'hydroquinone à utiliser dans une quantité 4 à 6 fois molaire, par rapport à la totalité de l'hydroquinone, d'acide chlorhydrique en solution aqueuse à 20-37 % déjà en place, et contenant des quantités catalytiques d'ions fer(III) et un dispersant anionique, on introduit dans cette solution, à une température de 20 à 90°C, une quantité 1,5 à 2,0 fois molaire, par rapport à la totalité de l'hydroquinone, de chlore gazeux, puis on ajoute la quantité restante d'hydroquinone sous forme solide ou dissoute, on introduit une quantité 1,5 à 2,0 fois molaire de chlore gazeux, en maintenant alors, par addition d'eau, la concentration de l'acide chlorhydrique d'abord à une valeur de 25 à 31 %, puis, en poursuivant l'insufflation de chlore gazeux (en une quantité 1,3 à 1,9 fois molaire), et tout en diluant avec de l'eau jusqu'à une concentration del'acide chlorhydrique de 20 à 22 %, on élève la température jusqu'à une valeur de 100 à 107°C, puis, après la fin de l'insufflation de chlore, on ajoute un solvant organique, ayant un point d'ébullition plus haut et non-miscible à l'acide chlorhydrique, en une quantité de 0,15 à 1,0 partie par partie de tétrachloro-1,4-benzoquinone, ou en une quantité d'environ 2 à 10 % en poids par rapport au mélange réactionnel, puis on soumet le mélange réactionnel à un post-traitement thermique.

4. Procédé selon au moins l'une des revendications 1 à 3, caractérisé en ce qu'on a en place dans le réacteur de 30 à 70 %, de préférence de 40 à 60 % et de préférence de 45 à 55 % de l'hydroquinone à utiliser.

5. Procédé selon au moins l'une des revendications 1 à 4, caractérisé en ce qu'on utilise un acide chlorhydrique aqueux à 22-33 %, en particulier à 31 %.

6. Procédé selon au moins l'une des revendications 1 à 4, caractérisé en ce qu'on utilise un acide de recyclage à environ 20 à 22 %.

7. Procédé' selon au moins l'une des revendications 1 à 6, caractérisé en ce qu'on procède à une insufflation de chlore à une température de 40 à 90°C et en particulier de 50 à 80°C.

8. Procédé selon au moins l'une des revendications 1 à 7, caractérisé en ce que le reste de l'hydroquinone est ajouté sous forme d'une solution aqueuse à 10-20 % et en particulier à 15-18 %.

9. Procédé selon au moins l'une des revendications 1 à 8, caractérisé en ce que la chloration est mise en oeuvre en un laps de temps de 5 à 15 heures et en particulier de 8,5 à 10,5 heures.

10. Procédé selon au moins l'une des revendications 1 à 9, caractérisé en ce que, après la fin de l'insufflation de chlore, on ajoute le solvant organique non-miscible à l'acide chlorhydrique en une quantité de 0,2 à 0,5 partie par partie de tétrachlorobenzoquinone ou en une quantité de 2 à 4 % en poids par rapport au mélange réactionnel.

11. Procédé selon au moins l'une des revendications 1 à 10, caractérisé en ce qu'on utilise en tant que solvant un hydrocarbure aromatique, en particulier le xylène, le toluène ou le diisopropylnaphtalène.

12. Procédé selon au moins l'une des revendications 1 à 11, caractérisé en ce que le post-traitement thermique est réalisé à une température de 90 à 100°C sous la pression normale.
